(19) **Europäisches Patentamt**
**European Patent Office**
**Office européen des brevets**

(11) **EP 3 692 596 B1**

(12) **FASCICULE DE BREVET EUROPEEN**

(45) Date de publication et mention de la délivrance du brevet:
**30.08.2023 Bulletin 2023/35**

(21) Numéro de dépôt: **18799578.2**

(22) Date de dépôt: **04.10.2018**

(51) Classification Internationale des Brevets (IPC):
*H01Q 1/27* (2006.01)    *A61J 3/00* (2006.01)
*H01Q 1/38* (2006.01)    *H01Q 9/04* (2006.01)
*H01Q 9/42* (2006.01)    *H01Q 21/28* (2006.01)
*H01Q 21/30* (2006.01)    *H01Q 5/371* (2015.01)

(52) Classification Coopérative des Brevets (CPC):
**H01Q 1/273; A61J 3/007; H01Q 1/38; H01Q 5/371; H01Q 9/0421; H01Q 9/42; H01Q 21/28; H01Q 21/30**

(86) Numéro de dépôt international:
**PCT/FR2018/052444**

(87) Numéro de publication internationale:
**WO 2019/069026 (11.04.2019 Gazette 2019/15)**

(54) **ANTENNE RADIOELECTRIQUE A BAS PROFIL MULTI-BANDE**

FLACHE MEHRBAND-FUNKANTENNE

LOW PROFILE MULTI-BAND RADIO ANTENNA

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **04.10.2017 FR 1759268**

(43) Date de publication de la demande:
**12.08.2020 Bulletin 2020/33**

(73) Titulaires:
• **Bodycap**
  **14200 Herouville Saint Clair (FR)**
• **Centre National de la Recherche Scientifique (CNRS)**
  **75016 Paris (FR)**
• **Université de Rennes 1**
  **35065 Rennes Cedex (FR)**

(72) Inventeurs:
• **NIKOLAYEV, Denys**
  **35700 Rennes (FR)**
• **ZHADOBOV, Maxim**
  **35830 Betton (FR)**
• **SAULEAU, Ronan**
  **35690 Acigné (FR)**

(74) Mandataire: **Fidal Innovation**
  **4-6 avenue d'Alsace**
  **92400 Courbevoie (FR)**

(56) Documents cités:
WO-A1-2011/111008    FR-A1- 2 703 872
KR-A- 20140 119 606    US-A1- 2004 263 396
US-A1- 2008 042 916    US-A1- 2012 280 885
US-A1- 2014 152 514    US-B1- 6 515 629

**Description**

DOMAINE TECHNIQUE

La présente description concerne une antenne radioélectrique multi-bande et un dispositif biotélémétrique équipé d'une telle antenne.

ETAT DE L'ART

Dans le domaine des applications médicales, les dispositifs biotélémétriques sont utilisés pour l'acquisition de signaux physiologiques et l'analyse des données physiologiques associées.

Parmi les dispositifs biotélémétriques, on trouve des dispositifs biotélémétriques ingestibles et/ou implantables *in vivo,* qui peuvent servir à la fois à la collecte de signaux physiologiques et la mise en oeuvre de fonctions thérapeutiques, comme par exemple la délivrance de médicaments ou la stimulation électrique. Ces dispositifs biotélémétriques se présentent par exemple sous la forme de capsules ingestibles ou d'implants pouvant être insérés dans le corps d'humains ou d'animaux.

Ces dispositifs biotélémétriques intègrent une antenne radioélectrique configurée pour émettre et/ou recevoir une onde électromagnétique. L'antenne radioélectrique est utilisée pour transmettre des données vers un équipement extérieur de contrôle / d'analyse des données ou pour recevoir les instructions émis par cet équipement. Ces équipements externes sont utilisés notamment pour analyser *ex vivo* les données transmises par l'antenne radioélectrique.

Les performances de l'antenne, en ce qui concerne la transmission de données et la puissance d'émission, dépendent fortement des propriétés électromagnétiques du milieu environnant.

En effet, lorsque le dispositif biotélémétrique est placé dans un milieu environnant (par exemple un milieu biologique), l'antenne est fortement couplée avec ce milieu environnant et l'impédance de l'antenne radioélectrique est ainsi fortement dépendante des propriétés électromagnétiques (permittivité, conductivité) de ce milieu environnant. Ainsi, une désadaptation d'impédance de l'antenne radioélectrique par rapport au microcontrôleur peut se produire du fait des propriétés électromagnétiques du milieu environnant.

Une désadaptation d'impédance entre l'antenne et le microprocesseur induit en effet des pertes d'énergie dans le dispositif biotélémétrique et impacte les performances en transmission de l'antenne, notamment la puissance de l'onde électromagnétique émise. Ceci est d'autant plus problématique que l'onde électromagnétique doit, pour parvenir à l'équipement externe, traverser des tissus biologiques humains ou animaux variés, qui sont fortement dispersifs, ce qui contribuent à la faible efficacité de rayonnement ($\eta$ < 0,1%).

En outre, lorsque l'impédance de l'antenne est affectée par le milieu environnant, cela se traduit également par une modification à la fois de la fréquence de résonance de cette antenne et son niveau d'adaptation à la fréquence de fonctionnement, ce niveau d'adaptation étant caractérisé par le coefficient de réflexion $|S_{11}|$ déterminé comme le rapport complexe (i.e. déterminé en amplitude et phase) entre l'intensité complexe du signal électrique incident converti par l'antenne en onde électromagnétique et l'intensité complexe du signal électrique réfléchi résultant d'une réflexion d'une fraction du signal électrique incident.

Une approche pour augmenter l'immunité de l'antenne à la désadaptation d'impédance est de concevoir une antenne radioélectrique à large bande, de sorte que la bande passante couvre la plage de fréquences dans laquelle l'antenne va fonctionner compte tenu de l'effet de désadaptation attendu. Cependant, une telle extension de la bande passante implique souvent de sacrifier l'efficacité de rayonnement.

L'article intitulé « Design, realization and measurements of a miniature antenna for implantable wireless communication systems », par F. Merli et al., IEEE Transactions Antennas Propagation, vol. 59, n°10, pages 3544-3555, octobre 2011, divulgue un exemple d'antenne radioélectrique multi-bande, sous forme hélicoïdale, ayant des dimensions appropriées pour une implantation sous-cutanée (10 $\times$ 32,1 mm). Cette antenne est toutefois lourde et volumineuse (elle occupe environ un quart du volume disponible) pour une intégration dans un dispositif biotélémétrique de type capsule ingestible ou implantable. Il est en effet difficile de produire une antenne miniature, adaptée pour une intégration dans un dispositif biotélémétrique, tout en améliorant les performances de transmission.

Le brevet US 6,515,629 divulgue une antenne du type F inversée à double bande comprenant un substrat une première ligne métallique rayonnante située sur une première surface du substrat, une deuxième ligne métallique rayonnante située sur la première surface du substrat; une ligne d'alimentation située sur la première surface du substrat et la ligne d'alimentation connectée aux positions médianes de la première ligne métallique rayonnante et de la seconde ligne métallique rayonnante pour un signal d'alimentation; une ligne de connexion située sur la première surface du substrat et utilisée pour se connecter à la première ligne métallique rayonnante et à la seconde ligne métallique rayonnante en même temps; et une broche métallique de court-circuit commune utilisée pour court-circuiter la première ligne métallique rayonnante et la seconde ligne métallique rayonnante vers un plan de masse.

La demande de brevet US 2012/0280885 divulgue une antenne comprenant deux éléments d'antenne et un circuit diviseur auquel les éléments d'antenne sont couplés via des lignes de transmission séparées. Un délai est mis en oeuvre sur une ligne de transmission en modifiant les longueurs des lignes de transmission couplant les éléments d'antenne au circuit diviseur, afin d'ajuster l'impédance et/ou la phase d'entrée d'un élément d'antenne.

La demande de brevet KR 2014 0119606A divulgue une antenne capsule comprenant un substrat, un résonateur à structure hélicoïdale et un plan de masse formés sur une même face de substrat, le plan de masse étant espacé du résonateur d'une distance prédéterminée.

La demande de brevet US2008/042916A1porte sur une antenne radiofréquence, pouvant notamment être mise en oeuvre dans un téléphone portable et présentant une pluralité de fréquences de résonance.

La demande de brevet US10/690,595, publiée sous le numéro US2004/0263396 porte sur une antenne radiofréquence présentant une pluralité de fréquences de résonance, pouvant notamment être mise en oeuvre dans un téléphone portable. L'antenne est de taille réduite par rapport aux antennes de l'art antérieur. La distorsion et la détérioration des caractéristiques due à l'influence du corps de l'utilisateur sont elles aussi réduites.

La demande de brevet FR9304353 concerne un applicateur à structure plane ou cylindrique à microrubans pour l'émission ou la réception de signaux haute-fréquence. Un tel applicateur est adapté pour des applications endocavitaires, notamment pour la mesure de température centrale par radiométrie micro-onde.

La demande de brevet internationale WO2011/111008 porte sur un dispositif de télémétrie comprenant un capteur et adapté pour des milieux à pertes, comprenant notamment une antenne double-bande.

Il apparaît ainsi un besoin de disposer d'une antenne multi-bande, robuste en impédance et adaptable en impédance pour au moins deux fréquences de résonance, qui soit peu volumineuse de sorte à être utilisable dans un dispositif biotélémétrique ingestible et/ou implantable in vivo.

RESUME

La présente description a pour objet, selon un premier aspect, une antenne radioélectrique multi-bande. Cette antenne radioélectrique comprend : un substrat formé en un matériau diélectrique ; un plan de masse en matériau électriquement conducteur, agencé sur une première face du substrat ; un résonateur configuré pour convertir un signal électrique incident en une onde électromagnétique et pour résonner à au moins deux fréquences de résonance distinctes, le résonateur comprenant au moins trois éléments agencés sur une deuxième face du substrat opposée à la première face. Un premier élément est formé par une première bande en matériau conducteur électriquement. Un deuxième élément est formé par une deuxième bande en matériau conducteur électriquement. Un troisième élément est formé par une troisième bande en matériau conducteur électriquement. Le deuxième élément est relié électriquement au plan de masse au moyen d'un via traversant le substrat au niveau d'une première extrémité de la deuxième bande. Le deuxième élément forme une extension du premier élément et est relié électriquement directement au premier élément au niveau d'une deuxième extrémité de la deuxième bande opposée à la première extrémité. Le premier élément est séparé du troisième élément par une fente et relié électriquement au troisième élément au niveau d'une extrémité de la première bande et d'une extrémité de la troisième bande par l'intermédiaire d'une bande de transition en matériau conducteur électriquement traversant la fente. Le troisième élément est relié à une ligne d'alimentation électrique pour recevoir le signal électrique incident.

Selon l'invention, le premier élément et le deuxième élément forment un résonateur à saut d'impédance à quart d'onde, avec une transition d'impédance entre le premier élément et le deuxième élément ayant une impédance supérieure à celle du premier élément, et le troisième élément rayonne comme une antenne patch demi-onde.

L'antenne radioélectrique selon le premier aspect utilise des éléments sous forme bande de matériau conducteur électriquement, réalisée par exemple, selon la technologie dite de microruban (« microstrip » selon la terminologie anglosaxonne).

Cette antenne microruban multibande convient pour de multiples utilisations invivo en permettant la communication (échange de données notamment) avec un dispositif externe au travers d'un milieu à pertes tel que le corps humain, ayant des propriétés électromagnétiques variables, en même temps que la mesure d'un coefficient de réflexion.

Elle peut être conçue de sorte à avoir une impédance donnée (par exemple 50 $\Omega$), pour au moins deux fréquences de fonctionnement (ou fréquences de résonance), par exemple pour les fréquences de fonctionnement 434 MHz et 2.45 GHz.

Cette antenne radioélectrique est une antenne multi-bande, présentant à la fois un haut rendement et une bonne robustesse en impédance. Ces caractéristiques sont acquises par le découplage d'antenne de milieu environnant à pertes à l'aide de design microruban spécifique de l'antenne. En outre, l'antenne peut être chargée diélectriquement par un superstrat à haute permittivité de sorte à améliorer sa robustesse et son rendement. Les gains pouvant être obtenus sont supérieurs -20 dBi, et l'efficacité de radiation est de l'ordre de 2% et 1% obtenue respectivement pour les fréquences de fonctionnement 434 MHz et 2.45 GHz.

En faisant varier les dimensions des trois éléments du résonateur, on peut ajuster les deux fréquences de résonance aux cas d'application visés. Du fait de la configuration particulière des bandes, il est également possible de

# EP 3 692 596 B1

réaliser une adaptation d'impédance pour les deux fréquences de résonance de l'antenne.

L'impédance de l'antenne est en outre peu sensible aux variations du milieu environnant. Le coefficient de réflexion $S_{11}$ est tel que $|S_{11}| < $ -10 dB dans toute une gamme de milieux environnants.

Une telle antenne se prête à une réalisation dans un circuit imprimé et peut en outre être intégrée dans une capsule biotélémétrique (par exemple, une capsule biotélémétrique ingestible, de longueur 28 mm et de diamètre 9 mm).

Une telle antenne radioélectrique se prête également à une réalisation d'antenne radioélectrique miniature, par exemple d'épaisseur de substrat inférieure à 1 mm et de largeur / longueur de substrat inférieure à 3 cm et peut ainsi aisément être intégrée dans un dispositif biotélémétrique telle qu'une capsule biotélémétrique. Une telle antenne est une antenne à bas profil (« low profile » selon la terminologie anglo-saxonne), l'épaisseur de l'antenne étant négligeable par rapport à sa taille. Une telle antenne occupe un espace négligeable dans une capsule biotélémétrique, permet un degré de miniaturisation élevé tout en ayant une efficacité accrue en transmission.

Une telle antenne radioélectrique peut en outre être réalisée sur un substrat flexible, de sorte à pouvoir se conformer à la surface intérieure d'un dispositif biotélémétrique tel qu'une capsule.

Dans un ou plusieurs modes de réalisation de l'antenne radioélectrique selon le premier aspect, le premier élément et le deuxième élément sont configurés pour résonner à une première fréquence de résonance et le troisième élément est configuré pour résonner à une deuxième fréquence de résonance supérieure à la première fréquence de résonance.

Dans un ou plusieurs modes de réalisation de l'antenne radioélectrique selon le premier aspect, le rapport entre la largeur de la première bande et la largeur de la deuxième bande est de 2:1 à 100:1.

Dans un ou plusieurs modes de réalisation de l'antenne radioélectrique selon le premier aspect, l'une au moins des première, deuxième, troisième et quatrième bandes a une forme parallélépipédique, de serpentin ou une forme en zigzag.

Dans un ou plusieurs modes de réalisation de l'antenne radioélectrique selon le premier aspect, l'épaisseur du substrat est de 20 $\mu$m à 5 mm.

Les caractéristiques des différents modes de réalisation de l'antenne radioélectrique selon le premier aspect sont combinables entre elles.

La présente description a pour objet, selon un deuxième aspect, un dispositif biotélémétrique comprenant une antenne radioélectrique selon le premier aspect. Les caractéristiques, propriétés, avantages et/ou effets de l'antenne radioélectrique selon le premier aspect sont transposables directement au dispositif biotélémétrique selon le deuxième aspect.

Dans un ou plusieurs modes de réalisation du dispositif biotélémétrique selon le deuxième aspect, le substrat étant dans un matériau flexible et le dispositif biotélémétrique étant sous forme de capsule dans laquelle le substrat est roulé de sorte que la première face du substrat est orientée vers l'intérieur de la capsule et la deuxième face est orientée vers l'extérieur de la capsule.

Dans un ou plusieurs modes de réalisation du dispositif biotélémétrique selon le deuxième aspect, le substrat de l'antenne radioélectrique est un substrat polyimide flexible se conformant à la surface interne de la capsule.

Dans un ou plusieurs modes de réalisation du dispositif biotélémétrique selon le deuxième aspect, le substrat est dans un matériau rigide et de forme cylindrique, le dispositif biotélémétrique étant intégré dans une capsule dans laquelle l'antenne radioélectrique est placée que la première face du substrat soit tournée vers l'intérieur de la capsule et la deuxième face soit tournée vers l'extérieur de la capsule.

Les caractéristiques des différents modes de réalisation du dispositif biotélémétrique selon le deuxième aspect sont combinables entre elles.

La présente description a pour objet, selon un troisième aspect, un Réseau d'antennes comprenant deux antennes selon le premier aspect, les deux antennes étant disposées symétriquement l'une par rapport à l'autre et alimentée par une même ligne électrique d'alimentation. Les caractéristiques, propriétés, avantages et/ou effets de l'antenne radioélectrique selon le premier aspect sont transposables directement au réseau d'antennes selon le troisième aspect.

BREVE DESCRIPTION DES FIGS.

D'autres avantages et caractéristiques de la technique présentée ci-dessus apparaîtront à la lecture de la description détaillée ci-dessous, faite par référence aux FIGS. dans lesquelles :

- la FIG. 1 représente de manière schématique un dispositif biotélémétrique selon un exemple de réalisation ;
- la FIG. 2 représentent de manière schématique une antenne radioélectrique selon un exemple de réalisation ;

- la FIG. 3 représentent de manière schématique un réseau d'antennes comprenant plusieurs radioélectriques selon un exemple de réalisation ;

4

- la FIG. 4 représente de manière schématique un dispositif biotélémétrique selon un exemple de réalisation ;
- la FIG. 5 illustre des aspects du fonctionnement et des performances d'une antenne radioélectrique selon un exemple de réalisation ;
- les FIG. 6A et 6B illustrent des aspects du fonctionnement et des performances d'une antenne radioélectrique selon un autre exemple de réalisation ;
- les FIG. 7A et 7B illustrent des aspects du fonctionnement et des performances d'une antenne radioélectrique selon un exemple de réalisation.

Dans les différents modes de réalisation qui vont être décrits par référence aux FIGS., des éléments semblables ou identiques portent les mêmes références.

DESCRIPTION DETAILLEE

Les différents modes de réalisation et aspects décrits ci-dessous peuvent être combinés ou simplifiés de multiples manières. Seuls certains modes de réalisation d'exemples sont décrits en détail pour assurer la clarté de l'exposé mais ces exemples ne visent pas à limiter la portée générale des principes ressortant de cette description considérée dans son ensemble.

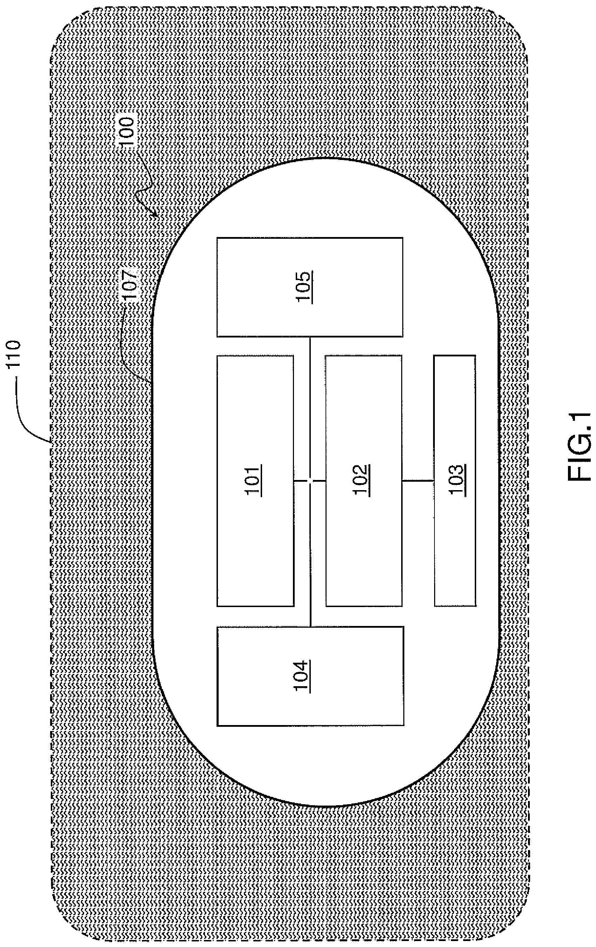

La FIG. 1 représente de manière schématique un exemple de dispositif biotélémétrique 100, sous forme de capsule implantable et/ou ingestible.

Le dispositif biotélémétrique 100 comprend un microcontrôleur 101, un circuit radiofréquence 102, une antenne radioélectrique 103, une source d'alimentation 104. De manière optionnelle, le dispositif biotélémétrique 100 peut comprendre un circuit additionnel 105, par exemple un circuit d'application biomédicale ou un capteur.

Dans un ou plusieurs modes de réalisation, la source d'alimentation 104 est configurée pour alimenter électriquement le microcontrôleur 101, le circuit radiofréquence 102, l'antenne radioélectrique 103 et le circuit additionnel 105.

Dans un ou plusieurs modes de réalisation, l'antenne radioélectrique 103 est configurée pour communiquer via une liaison radio avec un dispositif externe (non représenté), l'antenne radioélectrique 103 peut par exemple transmettre des données (par exemple des données de biotélémétrie acquises par le dispositif biotélémétrique 100) vers le dispositif externe et recevoir des données (par exemple des instructions opérationnelles et/ou de traitement thérapeutique) en provenance d'un tel dispositif externe.

Dans un ou plusieurs modes de réalisation, l'antenne radioélectrique peut émettre et recevoir des ondes électromagnétiques à fréquences élevées, par exemple dans la gamme du $10^8$ Hz à $10^{10}$ Hz.

Selon un ou plusieurs modes de réalisation, le microcontrôleur comprend une unité 112 de génération de signal électrique configurée pour générer le signal électrique incident. Selon un ou plusieurs modes de réalisation, le microcontrôleur comprend une unité 113 de traitement de données.

Dans un ou plusieurs modes de réalisation, le microcontrôleur 101 est configuré pour générer un signal électrique incident à convertir en onde électromagnétique par l'antenne radioélectrique et/ou pour amplifier un signal reçu en provenance de l'antenne radioélectrique.

Dans un ou plusieurs modes de réalisation, le microcontrôleur 101 est configuré pour traiter des données, par exemple pour traiter les données reçues par l'antenne radioélectrique 103 ou des données acquises par le circuit additionnel 105.

Dans un ou plusieurs modes de réalisation, l'ensemble des composants du dispositif biotélémétrique 100 (le microcontrôleur 101, le circuit radiofréquence 102, l'antenne radioélectrique 103, la source d'alimentation 104 et de manière optionnelle, le un circuit additionnel 105) est intégré dans une capsule biocompatible 107.

Dans un ou plusieurs modes de réalisation, le circuit radiofréquence 102 est interconnecté entre le microcontrôleur 101 et l'antenne radioélectrique 103. Le circuit radiofréquence 102 sert d'interface électrique entre le microcontrôleur 101.

Dans un ou plusieurs modes de réalisation, le circuit d'application biomédicale 105 est configuré pour mettre en oeuvre des fonctions de diagnostic et/ou des fonctions thérapeutiques. Les fonctions de diagnostic peuvent comprendre des fonctions d'acquisition ou mesure de données de diagnostic, par exemple au moyen d'un ou plusieurs capteurs, tels que par exemple, capteurs de température, capteurs électroniques, MEMS (« Microelectromechanical systems ») ou capteurs microfluidiques. Les fonctions de diagnostic peuvent comprendre des fonctions d'endoscopie, d'acquisition d'images, de mesure de glucose ou d'autres paramètres physiologiques, de détection d'anticorps, etc. Les fonctions thérapeutiques peuvent comprendre par exemple la délivrance de médicaments et la stimulation électrique, par exemple la stimulation cardiaque ou neurale.

Le dispositif biotélémétrique 100 est destiné à être utilisé dans un milieu environnant 110, par exemple après ingestion ou implantation *in vivo.* Au fur et à mesure que le dispositif biotélémétrique 100 se déplace dans le corps humain, par exemple pendant le transit gastro-intestinal, ce milieu environnant 110 est susceptible d'avoir des propriétés variées.

**EP 3 692 596 B1**

Les propriétés électromagnétiques (EM) du milieu environnant 110 entourant le dispositif biotélémétrique 100 détermine le couplage entre l'antenne radioélectrique 103 et le milieu environnant 110 et l'absorption des champs EM par ce milieu environnant 110. La connaissance de ces propriétés EM permet d'adapter la configuration de l'antenne radioélectrique 103 pour optimiser les performances de transmission sans fil de l'antenne radioélectrique 103 à travers le milieu environnant. En particulier, lorsque le couplage entre l'antenne radioélectrique 103 et le milieu environnant 110 est important, et les propriétés en transmission de l'antenne radioélectrique peuvent être affectées par les variations des propriétés EM du milieu environnant 110 dans lequel le dispositif biotélémétrique 100 se situe.

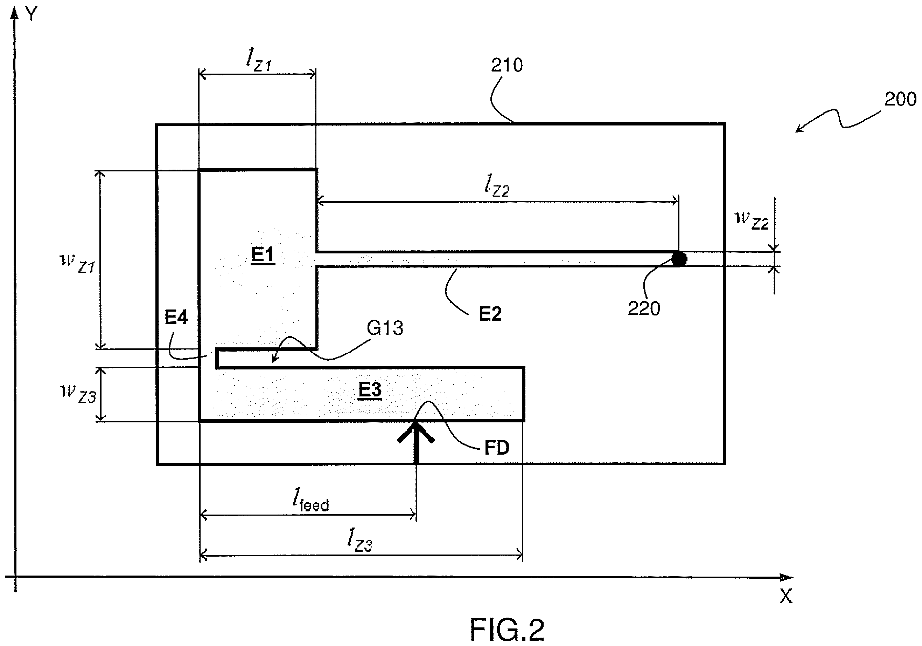

La FIG. 2 représente de manière schématique une configuration géométrique d'une antenne radioélectrique 200 adaptée pour un dispositif biotélémétrique 100 tel que celui décrit par référence à la FIG. 1, selon un exemple de réalisation.

Selon l'invention, les composants de l'antenne radioélectrique 200 sont intégrés dans un substrat 210 formé en un matériau diélectrique (par exemple, FR4, PFTE, polyimide, polyétheréthercétone, céramiques, composites, etc.). Le substrat 210 est constitué soit d'un matériau souple et/ou pouvant être roulé, de sorte à pouvoir se conformer à la surface intérieure d'un dispositif biotélémétrique tel qu'une capsule, soit d'un matériau rigide convenant pour la réalisation d'un dispositif biotélémétrique planaire. Le substrat est par exemple en polyimide flexible : un tel matériau est apte à se conformer à la surface interne d'une capsule de dispositif biotélémétrique.

Selon l'invention, un plan de masse est agencé sur une première face F2 (dessous) du substrat. Le plan de masse est par exemple réalisé en matériau électriquement conducteur (par exemple, en métal tel que cuivre, aluminium, argent etc. ou un alliage).

Selon l'invention, l'antenne radioélectrique 200 comprend un résonateur comprenant un premier élément E1, ayant une première impédance caractéristique Z1, un deuxième élément E2, ayant une deuxième impédance caractéristique Z2 et un troisième élément E3, ayant une troisième impédance caractéristique Z3.

Selon l'invention, le premier élément E1 est formé par une bande, en matériau conducteur électriquement, la bande étant agencée sur une deuxième face F1 (dessus) du substrat opposée à la première face F2. Le matériau du plan de masse peut être identique ou différent du matériau de la bande en matériau conducteur électriquement. La bande de matériau conducteur peut avoir différentes formes géométriques : une forme parallélépipédique, par exemple rectangulaire comme dans le cas d'exemple représenté à la FIG. 2, ou encore une autre forme (par exemple, serpentin, méandre, zigzag etc) de sorte à obtenir la longueur électrique et impédance souhaitée, notamment l'impédance qui satisfait les équations eqla et eqlb ci-dessous.

Selon l'invention, le deuxième élément E2 est formé par une bande, en matériau conducteur électriquement, la bande étant agencée sur la deuxième face F1. La bande de matériau conducteur peut avoir différentes formes géométriques : une forme parallélépipédique, par exemple rectangulaire comme dans le cas d'exemple représenté à la FIG. 2, ou encore une autre forme (par exemple, serpentin, méandre, zigzag etc) de sorte à obtenir la longueur électrique et impédance nécessaire pour satisfaire les équations eq1 et eqlb ci-dessous.

Selon l'invention, le troisième élément E3 est formé par une bande, en matériau conducteur électriquement, la bande étant agencée sur la deuxième face F1. La bande de matériau conducteur peut avoir différentes formes géométriques : une forme parallélépipédique, par exemple rectangulaire comme dans le cas d'exemple représenté à la FIG. 2, ou encore une autre forme (par exemple, serpentin, méandre ou zigzag, etc) de sorte à obtenir la longueur électrique qui satisfait sensiblement l'équation eq4 ci-dessous.

Les trois éléments E1, E2, E3 peuvent être dans un même matériau conducteur électrique ou dans différents matériaux conducteurs électriquement. Les matériaux utilisables sont par exemple le cuivre, l'aluminium, l'argent, un alliage, etc).

Selon l'invention, le troisième élément E3 est configuré pour recevoir un signal électrique incident généré par le microcontrôleur. Un point de connexion FD d'une ligne d'alimentation électrique est prévu sur le troisième élément E3. L'alimentation électrique peut être effectuée par microruban ou un câble coaxial. Dans un ou plusieurs modes de réalisation, le point d'alimentation électrique FD est situé sur un bord externe de la bande formant le troisième élément, ce bord externe étant opposé au côté de la troisième bande relié au premier élément E1 (voir l'exemple de la FIG. 2).

Selon l'invention, le deuxième élément E2 est relié électriquement au plan de masse, au niveau d'une première extrémité de la bande formant le deuxième élément, au moyen d'un via 220 traversant le substrat.

Selon l'invention, le deuxième élément E2 forme une extension du premier élément E1 et est relié électriquement directement au premier élément E1 au niveau d'une deuxième extrémité de la deuxième bande opposée à la première extrémité au niveau de laquelle se situe le via 220. Le premier élément E1 est ainsi relié électriquement au plan de masse par l'intermédiaire successivement du via 220 et du deuxième élément E2.

Selon l'invention, le premier élément E1 est séparé du troisième élément E3 par une fente G13 (« gap » selon la terminologie anglosaxonne). Dans un ou plusieurs modes de réalisation, la fente G13 est une fente rectiligne, de largeur fixe.

Selon l'invention, le premier élément E1 est relié électriquement au troisième élément E3 au niveau d'une extrémité de la première bande formant le premier élément E1 et d'une extrémité de la troisième bande formant le

6

troisième élément E3, par l'intermédiaire une bande de transition E4 en matériau conducteur électrique fermant la fente G13 à une de ses extrémités. Le troisième élément E3 est ainsi relié électriquement au plan de masse par l'intermédiaire successivement du via 220, du deuxième élément E2, du premier élément E1 et de la bande de transition E4.

Dans un ou plusieurs modes de réalisation, correspond à l'exemple illustré à la FIG. 2, la deuxième bande formant le deuxième élément E2 et la troisième bande formant le troisième élément E3 s'étendent longitudinalement selon une première direction (axe X dans la FIG. 2) et la première bande formant le premier élément E1 s'étend longitudinalement selon une deuxième direction (axe Y dans la FIG. 2), distincte de la première direction. Dans un ou plusieurs modes de réalisation, correspondant à l'exemple illustré à la FIG. 2, la première direction est perpendiculaire à la deuxième direction ou sensiblement perpendiculaire à la deuxième direction. Dans un ou plusieurs modes de réalisation, la première direction fait un angle compris entre 0° et 45° avec la deuxième direction.

Dans un ou plusieurs modes de réalisation, le résonateur présente au moins deux fréquences de résonance. Dans un ou plusieurs modes de réalisation, le résonateur est configuré pour convertir le signal électrique incident reçu par le troisième élément E3 en une onde électromagnétique à une fréquence correspondant à une des fréquences de résonance du résonateur. La fréquence de résonance est la fréquence pour laquelle la partie imaginaire de l'impédance complexe de l'antenne égale à zéro : $\text{Im}(Z_{\text{ANT}}) = 0$.

L'antenne présente au moins deux fréquences de résonance et deux bandes de fréquences correspondantes, ce qui augmente les possibilités d'utilisation de l'antenne. Par exemple, un transfert de données peut être réalisé dans une première bande de fréquences et un transfert d'énergie sans fil peut être réalisé dans une deuxième bande de fréquences (par exemple en utilisant la technique « Wireless Power Transfer » (WPT). Selon un autre exemple, une première bande de fréquences peut servir pour l'activation du dispositif dans lequel cette antenne est placée ainsi que pour la transmission de données, la deuxième bande pouvant servir pour la transmission de données en même temps que pour la transmission de données redondantes avec celles transmises via la première bande de sorte à pouvoir vérifier l'intégrité des données transmises via la première bande et ainsi augmenter la fiabilité de la transmission. En combinant dans une même antenne multi-bande la fonction de transmission de données et de transfert d'énergie, on peut sensiblement soit miniaturiser le dispositif dans lequel cette antenne est intégrée ou augmenter l'espace disponible à l'intérieur de ce dispositif.

La géométrie (notamment, les dimensions des trois éléments E1, E2, E3) de l'antenne radioélectrique satisfaisant la condition de résonance (chaque fréquence de résonance correspondant à une fréquence pour laquelle la partie imaginaire de l'impédance complexe de l'antenne $Z_{\text{ant}}$ est nulle : $\text{Im}(Z_{\text{ANT}}) = 0$) peut être déduite de l'équation d'impédance des lignes de transmission.

Les impédances caractéristiques Z1, Z2, Z3 des trois éléments E1, E2, E3 sont fonctions respectivement des dimensions de ces 3 éléments. Par référence à la FIG. 2, on note :

- $w_{Z1}$ la dimension selon l'axe Y du premier élément E1 ;
- $w_{Z2}$ la dimension selon l'axe Y du deuxième élément E2 ;
- $w_{Z3}$ la dimension selon l'axe Y du troisième élément E3 ;
- $w_{Z4}$ la dimension selon l'axe Y de la fente G13 et de la bande de transition E4 ;
- $l_{Z1}$ la dimension selon l'axe X du premier élément E1 ;
- $l_{Z2}$ la dimension selon l'axe X du deuxième élément E2 ;
- $l_{Z3}$ la dimension selon l'axe X du troisième élément E3 ; et
- $l_{\text{feed}}$ la position, par rapport à l'extrémité du troisième élément E3 (point origine de l'antenne), du point de connexion de la ligne d'alimentation électrique sur le bord externe de la troisième bande.

Dans un ou plusieurs modes de réalisation, le premier élément et le deuxième élément sont configurés pour résonner à une première fréquence de résonance et le troisième élément est configuré pour résonner à une deuxième fréquence de résonance supérieure à la première fréquence de résonance.

Selon l'invention, les éléments E1 et E2 fonctionnent comme un résonateur à saut d'impédance (SIR, pour « stepped impédance resonator ») à quart d'onde, avec une transition d'impédance (i.e. saut d'impédance) entre l'élément E1 à plus basse impédance Z1 et l'élément E2 à plus haute impédance Z2. En outre, un court-circuit (via 220) au plan de masse est réalisé à l'extrémité de l'élément E2 à plus haute impédance (extrémité haute impédance). Ceci implique une distribution de courant électrique dans les éléments présentant son minimum à l'extrémité de l'élément E1 à basse impédance Z1 (extrémité basse impédance) et son maximum à l'extrémité de l'élément E2 à haute impédance Z2 (extrémité haute impédance). La distribution de tension est inverse de celle du courant.

Les impédances caractéristiques Z1 et Z2 déterminent la fréquence de résonance la plus basse f1. Cette première fréquence de résonance f1 dépend principalement des impédances caractéristiques Z1, Z2 des premiers et deuxièmes éléments E1, E2 et est proportionnelle au rapport de ces impédances caractéristiques Z1 / Z2, tandis que la partie réelle de l'impédance d'antenne à cette première fréquence de résonance dépend des impédances caractéristiques Z1, Z2 des premiers et deuxièmes éléments E1 et E2 et est proportionnelle au produit de ces impédances caractéristiques Z1 × Z2.

En faisant varier le rapport des dimensions $w_{Z1}$ et $w_{Z2}$ des éléments E1, E2 tout en conservant la longueur totale de l'antenne $l = l_{Z1} + l_{Z2}$ fixe, on peut régler la première fréquence de résonance f1 à une valeur voulue (avec une infinité de possibilités pour Z1, Z2, $l_{Z1}$ et $l_{Z2}$) ainsi que l'impédance d'antenne souhaitée à cette première fréquence de résonance (par exemple 50 Ohm). Le rapport $w_{Z1}$ / $w_{Z2}$ peut par exemple varier entre 2:1 et 100:1.

Ainsi, il est possible d'ajuster la première fréquence de résonance f1 en choisissant de manière adaptée les dimensions selon l'axe Y des première et deuxième bandes, puis de faire varier les dimensions $w_{Z1}$, $w_{Z2}$ selon l'axe X des première et deuxième bandes, tout en gardant constant la longueur total de l'antenne, égale à la somme des dimensions $l_{Z1}$, $l_{Z2}$ selon l'axe X des première et deuxième bandes, de sorte à obtenir une impédance adaptée à la première fréquence de résonance souhaitée.

La première fréquence de résonance f1 est reliée aux dimensions selon l'axe Y des première et deuxième bandes par les relations suivantes :

$$- Z_1 + Z_2 \tan (\beta_{Z2}\, l_{Z2}) \tan(\beta_{Z1}\, l_{Z1}) = 0 \qquad \text{(eq1a)}$$

$$Z_2 \tan(\beta_{Z1}\, l_{Z1}) + Z_1 \tan(\beta_{Z2}\, l_{Z2}) \neq 0 \qquad \text{(eq1b)}$$

$\beta_{Z1}$ étant la constante de phase du premier élément E1 définie par :

$$\beta_{Z1} = \frac{2\pi}{c} f_1 \sqrt{\varepsilon_1^{r,eff}} \quad \text{(eq2)}$$

où $\beta_{Z2}$ est la constante de phase du deuxième élément E2 définie par

$$\beta_{Z2} = \frac{2\pi}{c} f_1 \sqrt{\varepsilon_2^{r,eff}} \quad \text{(eq3)}$$

c étant la vitesse de la lumière,

$\varepsilon_2^{r,eff}$ étant la permittivité relative effective du milieu environnant autour du deuxième élément,

$\varepsilon_1^{r,eff}$ étant la permittivité relative effective du milieu environnant autour du premier élément.

Selon l'invention, l'élément E3 rayonne comme une antenne patch demi-onde. Ceci implique une distribution de courant électrique présentant son minimum aux extrémités (selon l'axe X) de l'élément E3 et son maximum au milieu de l'élément E3. La distribution de tension est sinusoïdale avec des maxima aux extrémités (selon l'axe X) de l'élément E3 et son minimum au milieu de l'élément E3. L'élément E3 détermine les autres fréquences de résonances supérieures, notamment la deuxième fréquence de résonance f2. L'impédance Z3 de cet élément peut donc être choisie en fonction de la deuxième fréquence de résonance f2 visée.

La deuxième fréquence de résonance f2 dépend principalement de la dimension selon l'axe X du troisième élément E3 :

$$l_{Z3} \approx [2 \times \text{f2}\, (\varepsilon^{eff})^{0,5}\,]^{-1} \quad \text{(eq4)}$$

$\varepsilon^{eff}$ étant la permittivité effective du milieu environnant autour de l'élément E3. L'impédance de l'antenne $Z_{ANT}$ à cette deuxième fréquence de résonance dépend principalement de la dimension selon l'axe Y de la troisième bande $w_{Z3}$ et de la position $l_{feed}$ du point de connexion de la ligne d'alimentation électrique sur le bord externe de la troisième bande. L'impédance de l'antenne $Z_{ANT}$ à cette deuxième fréquence est notamment proportionnelle au rapport $1/w_{Z3}$. Ainsi, on peut ajuster la deuxième fréquence de résonance f2 à une valeur voulue et en même temps obtenir une impédance adaptée pour cette deuxième fréquence de résonance.

Dans un ou plusieurs modes de réalisation, une structure diélectrique (nommé superstrat) à faibles pertes et haute permittivité contribue à augmenter la permittivité effective $\varepsilon^{eff}$ (voir l'équation eq1 ci-dessus) et à découpler l'antenne du milieu environnant à pertes. Dans un ou plusieurs modes de réalisation, le superstrat diélectrique est disposé au-dessus des éléments E1, E2 et E3.

On peut par exemple réaliser une antenne radioélectrique présentant les fréquences de résonances f1= 434 MHz et f2 = 2,45 GHz, avec $w_{Z1}$ = 6.7 mm, $w_{Z2}$ = 140 μm, $w_{Z3}$ = 550 μm, $w_{Z4}$ = 200 μm, $l_{Z1}$ = 4.5 mm, $l_{Z2}$ = 13.5 mm, $l_{Z3}$ = 8 mm, $l_{feed}$ = 6 mm, un substrat d'épaisseur 102 μm en PEEK, un superstrat d'épaisseur 1 mm avec la permittivité relative $\varepsilon_r$ = 80, les 3 éléments E1, E2, E3 et la bande de transition E4 étant réalisés par un microruban en cuivre d'épaisseur 9 μm. Il a été montré qu'une telle antenne radioélectrique présente une immunité d'impédance dans les deux bandes ISM (industriel, scientifique et médical) autour de 434 MHz et 2,45 GHz. Pour les trois fréquences de résonance, une adaptation d'impédance à 50 Ohm est obtenue.

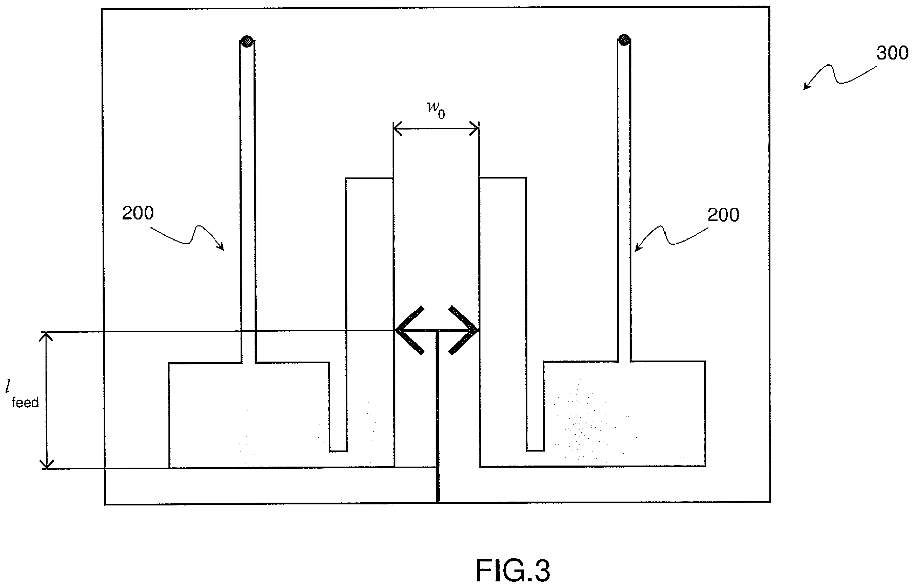

La FIG. 3 représente de manière schématique un réseau d'antennes 300 comprenant deux antennes selon la présente description sur un même substrat, les deux antennes étant disposées symétriquement l'une par rapport à l'autre et alimentée par une même ligne électrique d'alimentation. Dans un ou plusieurs modes de réalisation, la position $L_{feed}$ du point d'alimentation $L_{feed}$ des deux antennes est identique. Les deux antennes peuvent être identiques ou légèrement différentes, si on souhaite augmenter la bande passante. Le réseau peut comporter deux ou plusieurs antennes alimentées par une même ligne électrique d'alimentation.

Lorsque l'on utilise un tel réseau d'antenne dans un dispositif biotélémétrique sous forme de capsule, la configuration en réseau améliore l'omnidirectionnalité pour les fréquences de résonance les plus élevées. A la fréquence de résonance la plus basse, l'omnidirectionnalité de l'antenne est généralement suffisamment bonne. L'adaptation d'impédance par rapport aux composants du dispositif biotélémétrique (microprocesseur et circuit radiofréquence notamment) est réalisée en ajustant la distance W0 entre les éléments E3 de chaque antenne et en réglant la position $L_{feed}$ du point d'alimentation.

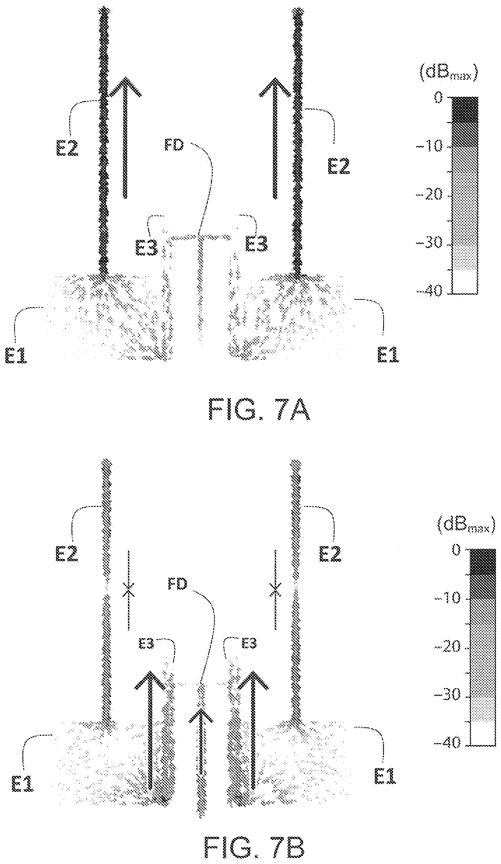

Les Figs. 7A et 7B représentent un exemple de distribution de courants pouvant être obtenue aux fréquences de résonance f1= 434 MHz (FIG. 7A) et f2= 2,45 GHz (FIG. 7B) respectivement avec un réseau de deux antennes selon la Fig. 3. Comme expliqué plus haut, le premier élément (E1) et le deuxième élément (E2) forment un résonateur à saut d'impédance à quart d'onde.

A la fréquence f1= 434 MHz (FIG. 7A), on observe des courants colinéaires dominants dans les éléments E2 avec un maximum sur les bords supérieurs où les vias 220 relient l'élément E2 au plan de masse. Dans ce cas, le saut d'impédance de $Z_1$ à Z2 définit la fréquence de résonance au moyen du ratio Zn= Z1/Z2 et des longueurs électriques $\beta_{Z1} l_{Z1}$ et $\beta_{Z2} l_{Z2}$. (voir équation eqla). La fréquence f1 choisie peut être obtenue pour une infinité de possibilités pour Z1, Z2, $l_{Z1}$ et $l_{Z2}$. Cependant, pour permettre un mode de fonctionnement simultanément à la fréquence f2= 2,45 GHz induit des contraintes supplémentaires sur pour Z1, Z2, $l_{Z1}$ et $l_{Z2}$.

A la fréquence f2= 2,45 GHz (FIG. 7B), l'onde émise par l'antenne provient principalement des courants colinéaires dans les éléments E3, avec une contribution venant de la ligne d'alimentation FD. On peut noter également une autre résonance au niveau de l'élément E2 : deux courants opposés ayant leur maximum d'intensité à l'extrémité la plus éloignée de l'élément E2 se rencontre au milieu (là où le minimum de courant est obtenu). Par contraste avec ce qui se passe à la fréquence 434 MHz, l'élément E1 se comporte comme un inverseur de phase à la fréquence f2= 2,45 GHz. En conséquence, le courant dans la moitié inférieure de l'élément E2 est colinéaire avec celui dans l'élément E3 : ceci contribue sensiblement à la radiation émise par l'antenne et impacte également l'impédance de l'antenne, en contribuant ainsi à l'adaptation de l'impédance de l'antenne par rapport au circuit radiofréquence.

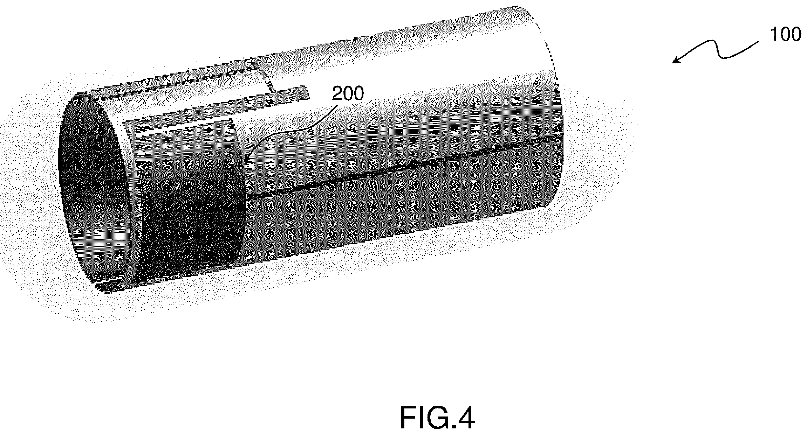

La FIG. 4 représente de manière schématique un dispositif biotélémétrique 100 comprenant au moins une antenne radioélectrique 200 ou un réseau d'antennes 300 selon la présente description dans un exemple de réalisation.

Le dispositif biotélémétrique est sous forme de capsule. La capsule peut être réalisée en matériau plastique biocompatible (PVC, PTFE, PEEK, Polyéthylène etc.), polymère ou céramique. La capsule est réalisée par exemple en céramique biocompatible, dont l'épaisseur est par exemple de 1 mm et la permittivité relative $\varepsilon_r$ = 80.

Dans un ou plusieurs modes de réalisation, le substrat est intégré dans la capsule de sorte que la face F2 du substrat sur laquelle est agencé le plan de masse soit tournée vers l'intérieur de la capsule et que l'autre face F1 sur laquelle sont agencés les trois éléments E1, E2, E3 sont tournés vers l'extérieur de la capsule. La taille de la capsule est par exemple comprise entre 15 mm et 50 mm de longueur et 5 mm à 15 mm de diamètre.

Dans un ou plusieurs modes de réalisation, le substrat de l'antenne radioélectrique est un substrat en matériau flexible, par exemple en polyimide flexible de 102 μm d'épaisseur se conformant à la surface interne de la capsule. L'épaisseur du substrat affecte principalement la bande passante d'impédance comme pour les antennes patch, en particulier à la fréquence de résonance F1 la plus faible. Plus généralement, l'épaisseur du substrat peut être comprise entre 20 μm et 3 mm.

Dans un ou plusieurs modes de réalisation, le substrat est dans un matériau rigide et de forme cylindrique de sorte à former une antenne radioélectrique 200 cylindrique. Les dimensions du substrat et le diamètre du cylindre formé par le substrat sont dans ce cas adaptés aux dimensions internes de la capsule avec la tolérance de 50 μm.

Dans un ou plusieurs modes de réalisation, un réseau d'antennes à deux antennes disposées en miroir comme celui illustré à la FIG. 3 est réalisé sur le substrat qui est ensuite intégré dans le dispositif biotélémétrique sous forme de capsule. Dans un exemple de réalisation, la première fréquence de résonance est 434 MHz et la deuxième fréquence de résonance est autour de 2.45 GHz lorsque le réseau d'antennes est utilisé dans un milieu avec les propriétés électromagnétiques équivalentes au tissu musculaire.

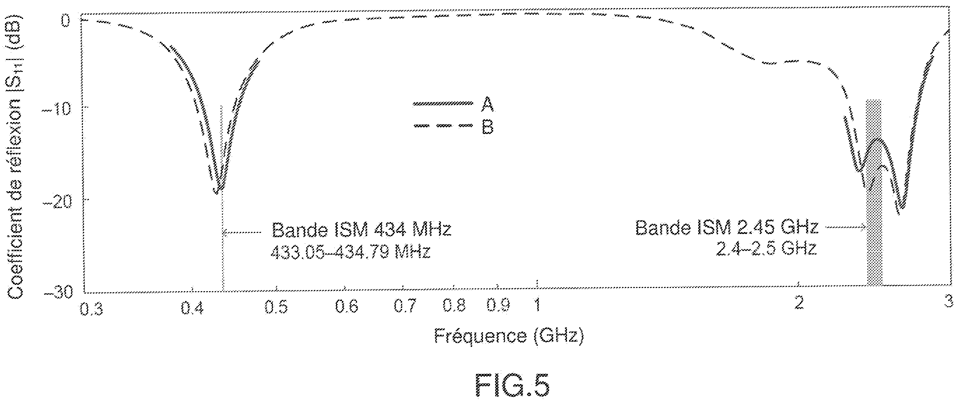

La FIG. 5 montre les performances d'une antenne en termes d'impédance selon un exemple de réalisation dans lequel l'antenne radioélectrique présente les propriétés pour les fréquences de résonances f1= 434 MHz (FIG. 5A) et f2 = 2,45 GHz (FIG. 5B), avec wzi = 6.7 mm, $w_{Z2}$ = 140 µm, $w_{Z3}$ = 550 µm, $w_{Z4}$ = 200 µm, $l_{Z1}$ = 4.5 mm, $l_{Z2}$ = 13.5 mm, $l_{Z3}$ = 8 mm, $l_{feed}$ = 6 mm, un substrat d'épaisseur 102 µm en PEEK, un superstrat d'épaisseur 1 mm avec la permittivité relative $\varepsilon_r$ = 80, les 3 éléments E1, E2, E3 et la bande de transition E4 étant réalisés par un microruban en cuivre d'épaisseur 9 µm. Les courbes de la FIG. 5 montrent la variation du coefficient de réflexion de l'antenne en fonction de la fréquence de fonctionnement. Les courbes A et B ont été obtenues respectivement par deux méthodes distinctes. Le gain réalisé est de -16,1 dBi à 434 MHz et -14,4 dBi à 2,45 GHz.

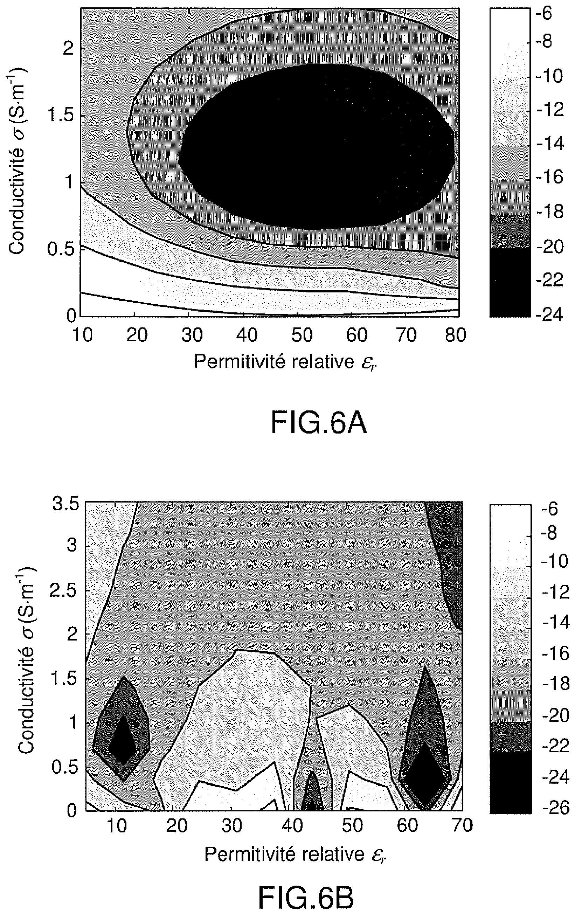

Les FIGS. 6A-6B montrent des courbes de variations du coefficient de réflexion $|S_{11}|$ en fonction de la conductivité et de la permittivité relative obtenues pour la même antenne que celle des FIGS. 5A et 5B pour les fréquences de résonances f1= 434 MHz (FIG. 6A) et f2 = 2,45 GHz (FIG. 6B). Ces FIGS. 6A-6B montrent la robustesse de l'antenne et sa capacité à fonctionner dans des milieux très variés (de conductivité entre 0 et 2,5 S/m et de permittivité relative entre 10 et 80) tout en conservant un coefficient de réflexion inférieur à - 10dB pour tous ces milieux. Ces milieux peuvent notamment être des tissus humains.

L'antenne radioélectrique décrite dans ce document peut être conçue pour fonctionner dans une large gamme de fréquences de résonance, par exemple entre $10^8$ Hz et $6 \times 10^{10}$ Hz. Une telle antenne qui est robuste aux variations des différentes propriétés EM du milieu environnant peut être utilisée dans n'importe quel tissu corporel pour de multiples scénarios d'application et présente de nombreuses possibilités d'application que ce soit dans le domaine médical ou non médical, par exemple, pour le génie civil, l'agriculture, la transformation des aliments, etc.

Une application est son utilisation dans un dispositif biotélémétrique ingestible et/ou implantable *in vivo* pour des applications de biotélémétrie et de téléthérapie dans le corps humain et/ou animal. Une autre application émergente est celle des dispositifs sans fil pour l'interfaçage neuronal bidirectionnel.

**Revendications**

1. Antenne radioélectrique multi-bande, l'antenne radioélectrique comprenant :

   - un substrat formé en un matériau diélectrique ;
   - un plan de masse en matériau électriquement conducteur, agencé sur une première face du substrat ;
   - un résonateur configuré pour convertir un signal électrique incident en une onde électromagnétique et pour résonner à au moins deux fréquences de résonance distinctes, le résonateur comprenant au moins trois éléments (E1, E2, E3) agencés sur une deuxième face du substrat opposée à la première face ;

   dans laquelle

   un premier élément (E1) est formé par une première bande en matériau conducteur électrique ; un deuxième élément (E2) est formé par une deuxième bande en matériau conducteur électrique ; un troisième élément (E3) est formé par une troisième bande en matériau conducteur électrique ;
   le deuxième élément (E2) est relié électriquement au plan de masse au moyen d'un via (220) traversant le substrat au niveau d'une première extrémité de la deuxième bande ;
   le deuxième élément (E2) forme un extension du premier élément (E1) et est relié électriquement directement au premier élément (E1) au niveau d'une deuxième extrémité de la deuxième bande opposée à la première extrémité ;
   le premier élément (E1) est séparé du troisième élément (E3) par une fente (G13) et relié électriquement au troisième élément (E3) au niveau d'une extrémité de la première bande et d'une extrémité de la troisième bande par l'intermédiaire d'une bande de transition (E4) en matériau conducteur électriquement traversant la fente ;
   le troisième élément (E3) est relié à une ligne d'alimentation électrique pour recevoir le signal électrique incident ;
   le premier élément (E1) et le deuxième élément (E2) sont configurés de manière à former un résonateur à saut d'impédance à quart d'onde, avec un saut d'impédance entre le premier élément (E1) et le deuxième élément (E2) ayant une impédance supérieure à celle du premier élément (E1); et
   le troisième élément (E3) est configuré pour rayonner comme une antenne patch demi-onde.

2. Antenne selon la revendication précédente dans laquelle le premier élément (E1) et le deuxième élément (E2) sont configurés pour résonner à une première fréquence de résonance et le troisième élément (E3) est configuré pour résonner à une deuxième fréquence de résonance supérieure à la première fréquence de résonance.

3. Antenne selon l'une quelconque des revendications précédentes dans laquelle le rapport entre la largeur de la première bande et la largeur de la deuxième bande est de 2:1 à 100:1.

4. Antenne selon l'une quelconque des revendications précédentes, dans laquelle l'une au moins des première, deuxième, troisième et quatrième bandes a une forme parallélépipédique, de serpentin ou une forme en zigzag.

5. Antenne selon l'une quelconque des revendications précédentes, dans laquelle l'épaisseur du substrat est de 20 $\mu$m à 5 mm.

6. Antenne selon l'une quelconque des revendications précédentes, dans laquelle la fente (G13) est une fente rectiligne et de largeur sensiblement fixe.

7. Antenne selon l'une quelconque des revendications précédentes, comprenant un superstrat à haute permittivité.

8. Antenne selon la revendication 7, dans laquelle le superstrat est disposé au-dessus des premier, deuxième et troisième éléments.

9. Antenne selon l'une quelconque des revendications précédentes, dans laquelle le premier élément (E1) s'étend longitudinalement selon une direction Y distincte de la première direction X dans laquelle s'étend longitudinalement le deuxième élément (E2) ; et la fréquence de résonance f1 du résonateur à saut d'impédance est reliée aux dimensions $l_{Z1}$ et $l_{Z2}$ selon la direction Y des première et deuxième bandes par les équations suivantes :

$$- Z_1 + Z_2 \tan \left(\beta_{Z2}\, l_{Z2}\right) \tan(\beta_{Z1}\, l_{Z1}) = 0$$

$$Z_2 \tan(\beta_{Z1}\, l_{Z1}) + Z_1 \tan(\beta_{Z2}\, l_{Z2}) \neq 0$$

où

Z1 est l'impédance du premier élément (E1),
Z2 est l'impédance du deuxième élément (E2),
$\beta_{Z1}$ étant la constante de phase du premier élément (E1) définie par :

$$\beta_{Z1} = \frac{2\pi}{c} f_1 \sqrt{\varepsilon_1^{r,eff}}$$

$\beta_{Z2}$ est la constante de phase du deuxième élément (E2) définie par :

$$\beta_{Z2} = \frac{2\pi}{c} f_1 \sqrt{\varepsilon_2^{r,eff}}$$

c étant la vitesse de la lumière,
$\varepsilon_2^{r,eff}$ étant la permittivité relative effective du milieu environnant autour du deuxième élément (E2),
$\varepsilon_1^{r,eff}$ étant la permittivité relative effective du milieu environnant autour du premier élément (E1).

10. Réseau d'antennes comprenant deux antennes selon l'une quelconque des revendications précédentes, les deux antennes étant disposées symétriquement l'une par rapport à l'autre et alimentée par une même ligne électrique d'alimentation.

11. Dispositif biotélémétrique comprenant une antenne radioélectrique selon l'une quelconque des revendications 1 à 9.

**12.** Dispositif biotélémétrique selon la revendication 11, le substrat étant dans un matériau flexible et le dispositif biotélémétrique étant sous forme de capsule dans laquelle le substrat est roulé de sorte que la première face (F2) du substrat est orientée vers l'intérieur de la capsule et la deuxième face (F1) est orientée vers l'extérieur de la capsule.

**13.** Dispositif biotélémétrique selon la revendication 12, dans lequel le substrat de l'antenne radioélectrique est un substrat polyimide flexible se conformant à la surface interne de la capsule.

**14.** Dispositif biotélémétrique selon la revendication 11, dans lequel le substrat est dans un matériau rigide et de forme cylindrique, le dispositif biotélémétrique étant intégré dans une capsule dans laquelle l'antenne radioélectrique est placée que la première face du substrat soit tournée vers l'intérieur de la capsule et la deuxième face soit tournée vers l'extérieur de la capsule.


**Patentansprüche**

**1.** Mehrband-Funkantenne, wobei die Funkantenne Folgendes umfasst:

- ein Substrat, das aus einem dielektrischen Material gebildet ist;
- eine Masseebene aus elektrisch leitfähigem Material, die auf einer ersten Seite des Substrats angeordnet ist;
- einen Resonator, der zum Umwandeln eines einfallenden elektrischen Signals in eine elektromagnetische Welle und zum Resonieren bei mindestens zwei verschiedenen Resonanzfrequenzen konfiguriert ist, wobei der Resonator mindestens drei Elemente (E1, E2, E3) umfasst, die auf einer zweiten Seite des Substrats gegenüber der ersten Seite angeordnet sind;

wobei

ein erstes Element (E1) durch ein erstes Band aus elektrisch leitfähigem Material gebildet ist; ein zweites Element (E2) durch ein zweites Band aus elektrisch leitfähigem Material gebildet ist; ein drittes Element (E3) durch ein drittes Band aus elektrisch leitfähigem Material gebildet ist;

das zweite Element (E2) mittels einer das Substrat an einem ersten Ende des zweiten Bandes durchquerenden Durchkontaktierung (220) mit der Masseebene elektrisch verbunden ist;

das zweite Element (E2) eine Verlängerung des ersten Elements (E1) bildet und an einem dem ersten Ende gegenüberliegenden zweiten Ende des zweiten Bandes elektrisch direkt mit dem ersten Element (E1) verbunden ist;

das erste Element (E1) durch einen Schlitz (G13) vom dritten Element (E3) getrennt und an einem Ende des ersten Bandes und an einem Ende des dritten Bandes über ein durch den Schlitz verlaufendes Übergangsband (E4) aus elektrisch leitendem Material elektrisch mit dem dritten Element (E3) verbunden ist;

das dritte Element (E3) mit einer Stromversorgungsleitung verbunden ist, um das einfallende elektrische Signal zu empfangen;

das erste Element (E1) und das zweite Element (E2) zum Bilden eines Viertelwellen-Impedanzsprungresonators konfiguriert sind, mit einem Impedanzsprung zwischen dem ersten Element (E1) und dem zweiten Element (E2), das eine höhere Impedanz als das erste Element (E1) aufweist; und

das dritte Element (E3) zum Strahlen wie eine Halbwellen-Patch-Antenne konfiguriert ist.

**2.** Antenne nach dem vorherigen Anspruch, wobei das erste Element (E1) und das zweite Element (E2) zum Resonieren bei einer ersten Resonanzfrequenz konfiguriert sind und das dritte Element (E3) zum Resonieren bei einer zweiten Resonanzfrequenz konfiguriert ist, die höher als die erste Resonanzfrequenz ist.

**3.** Antenne nach einem der vorherigen Ansprüche, wobei das Verhältnis zwischen der Breite des ersten Bandes und der Breite des zweiten Bandes 2:1 bis 100: 1 beträgt.

**4.** Antenne nach einem der vorherigen Ansprüche, wobei mindestens eines von erstem, zweitem, drittem und viertem Band parallelepipedförmig, schlangenförmig oder zickzackförmig ist.

**5.** Antenne nach einem der vorherigen Ansprüche, wobei die Dicke des Substrats 20 µm bis 5 mm beträgt.

**6.** Antenne nach einem der vorherigen Ansprüche, wobei der Schlitz (G13) ein geradliniger Schlitz mit einer im Wesentlichen festen Breite ist.

7. Antenne nach einem der vorherigen Ansprüche, die ein Superstrat mit hoher Permittivität umfasst.

8. Antenne nach Anspruch 7, wobei das Superstrat über dem ersten, zweiten und dritten Element angeordnet ist.

9. Antenne nach einem der vorherigen Ansprüche, wobei sich das erste Element (E1) longitudinal in einer Richtung Y erstreckt, die sich von der ersten Richtung X unterscheidet, in der sich das zweite Element (E2) longitudinal erstreckt; und die Resonanzfrequenz f1 des Impedanzsprungresonators durch die folgenden Gleichungen mit den Abmessungen $l_{Z1}$ und $l_{z2}$ in der Richtung Y des ersten und zweiten Bandes verbunden ist:

$$- Z_1 + Z_2 \tan (\beta_{Z2}\ l_{Z2}) \tan (\beta_{Z1}\ l_{Z1}) = 0$$

$$Z_2 \tan(\beta_{Z1}\ l_{Z1}) + Z_1 \tan(\beta_{Z2}\ l_{Z2}) \neq 0$$

wobei

Z1 die Impedanz des ersten Elements (E1) ist,
Z2 die Impedanz des zweiten Elements (E2) ist,
$\beta_{Z1}$ die Phasenkonstante des ersten Elements (E1) ist, definiert durch:

$$\beta_{Z1} = \frac{2\pi}{c}\ f_1 \sqrt{\varepsilon_1^{r,eff}}$$

$\beta_{Z2}$ die Phasenkonstante des zweiten Elements (E2) ist, definiert durch :

$$\beta_{Z2} = \frac{2\pi}{c}\ f_1 \sqrt{\varepsilon_2^{r,eff}}$$

c die Geschwindigkeit des Lichts ist;
wobei $\varepsilon_2^{r,eff}$ die effektive relative Permittivität des Umgebungsmediums um das zweite Element (E2) ist,
wobei $\varepsilon_1^{r,eff}$ die effektive relative Permittivität des Umgebungsmediums um das erste Element (E1) ist.

10. Antennengruppe mit zwei Antennen nach einem der vorherigen Ansprüche, wobei die beiden Antennen symmetrisch zueinander angeordnet sind und von einer gemeinsamen elektrischen Versorgungsleitung gespeist werden.

11. Biotelemetrische Vorrichtung mit einer Funkantenne nach einem der Ansprüche 1 bis 9.

12. Biotelemetrische Vorrichtung nach Anspruch 11, wobei das Substrat aus einem flexiblen Material besteht und die biotelemetrische Vorrichtung die Form einer Kapsel hat, in die das Substrat so gerollt ist, dass die erste Seite (F2) des Substrats zum Inneren der Kapsel orientiert ist und die zweite Seite (F1) zum Äußeren der Kapsel orientiert ist.

13. Biotelemetrische Vorrichtung nach Anspruch 12, wobei das Substrat der Funkantenne ein flexibles Polyimidsubstrat ist, das sich an die Innenfläche der Kapsel anpasst.

14. Biotelemetrische Vorrichtung nach Anspruch 11, wobei das Substrat aus einem starren Material besteht und eine zylindrische Form hat, wobei die biotelemetrische Vorrichtung in eine Kapsel integriert ist, in der die Funkantenne so platziert ist, dass die erste Seite des Substrats dem Inneren der Kapsel zugewandt ist und die zweite Seite dem Äußeren der Kapsel zugewandt ist.

**Claims**

1. A multi-band radio antenna, wherein the radio antenna comprises

   - a substrate formed of a dielectric material;
   - a ground plane of electrically conductive material, arranged on a first face of the substrate;
   - a resonator configured to convert an incident electrical signal into an electromagnetic wave and to resonate at at least two distinct resonance frequencies, the resonator comprising at least three elements (E1, E2, E3) arranged on a second face of the substrate opposite to the first side;

   wherein

   a first element (E1) is formed by a first strip of electrically conductive material; a second element (E2) is formed by a second strip of electrically conductive material; a third element (E3) is formed by a third strip of electrically conductive material;
   the second element (E2) is electrically connected to the ground plane by means of a via (220) passing through the substrate at a first end of the second strip;
   the second element (E2) forms an extension of the first element (E1) and is electrically connected directly to the first element (E1) at a second end of the second strip opposite the first end;
   the first element (E1) is separated from the third element (E3) by a slot (G13) and electrically connected to the third element (E3) at one end of the first strip and one end of the third strip by means of a transition strip (E4) of electrically conductive material passing through the slot;
   the third element (E3) is connected to an electrical supply line to receive the incident electrical signal;
   the first element (E1) and the second element (E2) are configured to form a quarter-wave resonator with impedance jump, with an impedance jump between the first element (E1) and the second element (E2) having an impedance greater than that of the first element (E1);
   and the third element (E3) is configured to radiate like a half-wave patch antenna.

2. The antenna according to the preceding claim in which the first element (E1) and the second element (E2) are configured to resonate at a first resonance frequency and the third element (E3) is configured to resonate at a second resonance frequency greater than the first resonance frequency.

3. The antenna according to any one of the preceding claims in which the ratio of the width of the first strip to the width of the second strip is from 2 : 1 to 100 : 1.

4. The antenna according to any one of the preceding claims, in which at least one of the first, second, third and fourth strips has a parallelepipedic, coiled or zigzag shape.

5. The antenna according to any one of the preceding claims, in which the thickness of the substrate is from 20 $\mu$m to 5 mm.

6. The antenna according to any one of the preceding claims, in which the slot (G13) is a straight slot and of substantially fixed width.

7. The antenna according to any one of the preceding claims, comprising a high permittivity superstrate.

8. The antenna according to claim 7, in which the superstrate is arranged above the first, second and third elements.

9. The antenna according to any one of the preceding claims, in which the first element (E1) extends longitudinally along a direction Y distinct from the first direction X along which the second element extends longitudinally; and the resonance frequency f1 of the impedance jump resonator is related to the dimensions $l_{Z1}$ and $l_{Z2}$ along the direction Y of the first and second bands by the following equations :

$$- Z_1 + Z_2 \tan(\beta_{Z2}\, l_{Z2}\,) \tan(\beta_{Z1}\, l_{Z1}\,) = 0$$

$$Z_2 \tan(\beta_{Z1}\, l_{Z1}\,) + Z_1 \tan(\beta_{Z2}\, l_{Z2}\,) \neq 0$$

where $Z_1$ is the impedance of the first element,
$Z_2$ is the impedance of the second element,
$\beta_{Z1}$ being the phase constant of the first element defined by:

$$\beta_{Z1} = \frac{2\pi}{c} f_1 \sqrt{\varepsilon_1^{r,eff}}$$

$\beta_{Z2}$ is the phase constant of the second element defined by :

$$\beta_{Z2} = \frac{2\pi}{c} f_1 \sqrt{\varepsilon_2^{r,eff}}$$

c being the speed of light,

$\varepsilon_2^{r,eff}$ being the effective relative permittivity of the surrounding medium around the second element (E2),

$\varepsilon_1^{r,eff}$ being the effective relative permittivity of the surrounding medium around the first element (E1).

10. An antenna array comprising two antennas according to any one of the preceding claims, the two antennas being arranged symmetrically with respect to each other and supplied by the same power supply line.

11. A biotelemetry device comprising a radio antenna according to any one of claims 1 to 9.

12. A biotelemetry device according to claim 11, the substrate being in a flexible material and the biotelemetry device being in the form of a capsule in which the substrate is rolled so that the first face (F2) of the substrate faces the interior of the capsule and the second face (F1) faces the outside of the capsule.

13. A biotelemetry device according to claim 12, in which the substrate of the radio antenna is a flexible polyimide substrate conforming to the internal surface of the capsule.

14. The biotelemetry device according to claim 11, in which the substrate is made of a rigid material and cylindrically shaped, the biotelemetry device being integrated in a capsule in which the radioelectric antenna is placed so that the first face of the substrate faces the interior of the capsule and the second face faces the outside of the capsule.

FIG.1

EP 3 692 596 B1

FIG.2

EP 3 692 596 B1

FIG.3

EP 3 692 596 B1

FIG.4

FIG.5

FIG.6A

FIG.6B

FIG. 7A

FIG. 7B

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6515629 B
- US 20120280885 A
- KR 20140119606 A
- US 2008042916 A1
- US 10690595 B
- US 20040263396 A
- FR 9304353
- WO 2011111008 A

**Littérature non-brevet citée dans la description**

- **F. MERLI et al.** Design, realization and measurements of a miniature antenna for implantable wireless communication systems. *IEEE Transactions Antennas Propagation,* Octobre 2011, vol. 59 (10), 3544-3555